# EUROPEAN PATENT APPLICATION

(11) **EP 2 484 695 A1**
(43) Date of publication of application: **08.08.2012**
(21) Application number: 11000898.4
(22) Date of filing: 04.02.2011
(51) Int. Cl.: C07K 16/18

(54) **A method for the production of hybridoma cell lines producing monoclonal antibodies capable to specifically binding to a human C44-fragment of agrin**

(71) Applicant: Neurotune AG, 8952 Schlieren (CH)
(72) Inventor: Dahinden, Pius, CH-5412 Gebenstorf (CH); Hettwer, Stefan, CH-8051 Zürich (CH); Vrijbloed, Jan Willem, CH-4313 Möhlin (CH)
(74) Representative: Emmel, Thomas

(57) **Abstract**

A method for the production of a hybridoma cell lines producing monoclonal antibodies capable to specifically binding to a human C44-fragment of agrin, comprising administering to wild-type-mice an immunizing amount of C44y≥4-fragment of agrin, isolating antibody producing cells from the immunized mice, fusing them with a myeloma cell line, growing the fused cells in a selection medium, screening the antibodies in the supernatants of hybridoma cells for binding to C44-fragment of agrin and isolating the hybridoma cells producing the desired monoclonal antibodies

## Description

The present invention relates to a method for the production of hybridoma cell lines producing antibodies against C-terminal fragments of agrin, antibodies produced by the cell lines, pharmaceutical compositions containing said antibodies, and uses thereof.

Agrin is an important protein which plays a pivotal role in the synapse-formation process by assisting formation and maintenance of the postsynaptic apparatus of developing neuromuscular junctions (NMJ's) (Bezakova and Ruegg, 2003). It could be shown that agrin deficient mice die at birth due to respiratory failure. This is caused by the fact that agrin is strictly required for the proper innervation of muscle fibres and that these mice are not able to build proper NMJ's.

Agrin is not only existent in neural or neuronal tissues but can also be found in periphery tissues like the lung and the kidney which indicates that agrin plays also a role in these organs.

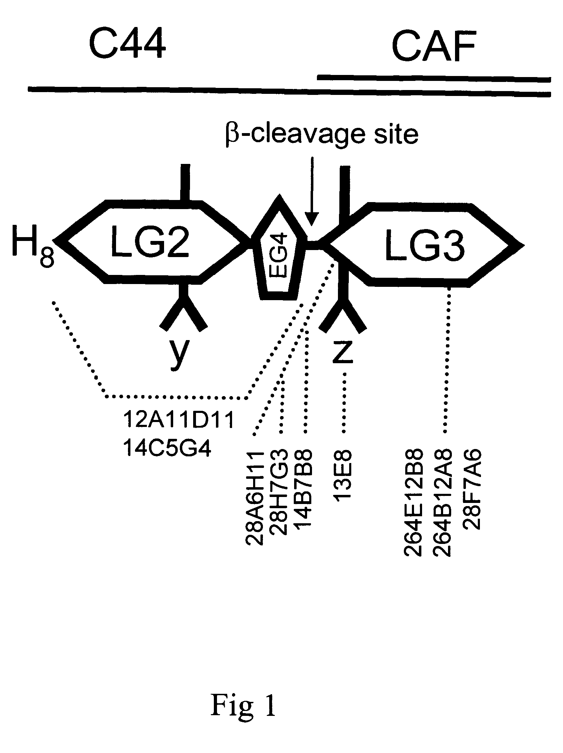

Agrin is a large heparan proteoglycan with a molecular weight of 400 - 600 kDa. (Database accession number NP_940978). The protein core consists of about 2000 amino acids and its mass is about 225 kDa. It is a multidomain protein composed of 9 K (Kunitz-type) domains, 2 LE (laminin-EGF-like) domains, one SEA (sperm protein, enterokinase and agrin) domain, 4 EG (epidermal growth factor-like) domains and 3 LG (laminin globular) domains (Fig. 1).

Agrin exists in several splice variants and can be expressed as a secreted protein, containing the N-terminal NtA (N-terminal agrin) domain, which is the most abundant form of agrin and the predominant form expressed in motor neurons. It is produced in the soma of the neurons, transported down the axon and released from the axon ending of the motor nerve into the synaptic cleft of the NMJ. Here it acts as an agonist of LRP4 (low-density lipoprotein receptor-related protein 4) and may also become a component of the basal lamina. In the CNS (central nervous system), most agrin is expressed as a type-II transmembrane protein by alternative splicing at the N-terminus lacking the N-terminal NtA domain (Bezakova and Ruegg, 2003).

In the C-terminal part of human agrin, there are 2 alternative splice sites y and z. At the y-site, there may be inserts of 0, 4, 17 or 21 (4+17) amino acids and at the z site there may be inserts of 0, 8, 11 or 19 (8+11) amino acids. The function of the four inserted amino acids in the y-site is to create a heparin binding site. Motor neurons express predominantly y4 agrin. The most important splice site of agrin in respect of NMJ maturation is the z-site, giving agrin the ability to be active as an acetylcholine-receptor clustering agent. It is well known that full length agrin containing the insertion of 8 amino acids at the z-site in presence of the 4 amino acid insert in splice site y (y4z8) generates an agrin variant with a half maximal AChR clustering activity of 35 pM in cultured myotube clustering assays. The insertion of 11 amino acids give rise to a half maximal AChR clustering activity of 5 nM while the 19 amino acid insertion results in a half maximal AChR clustering activity of 110 pM. Agrin without an insertion at this site is not active in clustering acetylcholine-receptors on the in-vitro cultured myotubes (Bezakova and Ruegg, 2003). Thus, the most active form of agrin in the clustering assay is the y4z8 variant, which is expressed by motor neurons.

A ~45 kDa C-terminal fragment of agrin (y4z8) containing the LG2, EGF4 and the LG3 domains was found to be active in AChR clustering with an EC50 of 130 pM in the AchR clustering activity while shorter fragments have only lower activities. The C-terminal LG3 domain with the z8 insertion exhibits a half maximal AChR clustering activity of only 13 nM, which is a factor 100 fold lower than the 45 kDa fragment (Bezakova and Ruegg, 2003).

In WO 97/21811 it is proposed to use agrin or agrin fragments in methods of treatment of a disease that affects muscle. However, such attempts have up to date not shown to be successful.

Investigations of the applicant have confirmed that in vivo activity of the agrin-fragment is mainly dependent on the presence of both domains LG2 and LG3 together. WO97/21811 showed in vitro activity for AChR-clustering for LG3 alone but no in vivo activity of LG3 could be shown. As it appears activation of LRP4 alone is not sufficient to achieve full in vivo activity.

Agrin is cleaved by an enzyme called neurotrypsin which plays an important role in controlling the activity of agrin. At present, agrin is the only known target of neurotrypsin. Neurotrypsin (Stephan A, et al: The FASEB Journal. 2008;22:1861-1873) cleaves agrin at 2 distinct sites called alpha-and beta-site. (Fig. 1). The alpha-site is located N-terminal from the SEA domain and the beta-site is placed in front of the LG3 domain of agrin. Cleavage at the alpha-site generates a ~110 kDa C-terminal agrin fragment running art ~130 kDa in a 4-12 % bis-tris SDS gel. Cleavage at the beta-site liberates the C-terminal LG3 domain running at ~22 kDa in the gel (Molinari, Rio et al., 2002; Reif, Sales et al., 2007). Cleavage at the beta-site leads to a separation of the LG2 domain from the LG3 domain.

It was found that neurotrypsin (NT) over-expressing mice, so-called sarcopenia mice (muslik, M491 S) (Stephan, Mateos et al., 2008), show an early onset of sarcopenia, a degenerative loss of skeletal muscle mass and strength associated with aging.

All C-terminal fragments could be detected in brain extracts and spinal cord extracts of mice (Stephan, Mateos et al., 2008). In neurotrypsin knock out mice, none of the fragments could be detected. As a consequence, neurotrypsin seems to be the only protease which cleaves agrin in significant amounts at the two cleavage sites. Cleavage of agrin by neurotrypsin can generate in principle five different agrin fragments, 3 of which can be detected in blood. These three agrin fragments CAF, C90 and C110. These fragments, also depending on the presence of specific inserts at the y and z position, can have different functions. A list with the various naturally occurring and artificial agrin fragments used in this patent are described in the table 1 below.

**Table 1**

| Abbreviation | Description |
|---|---|
| CAF | Naturally occurring 22kd C-terminal agrin fragment generated by Neurotrypsin cleavage of Agrin at the beta cleavage site. The 22-kD corresponds to the apparent running position on PAGE gel. Insert at the Z position is not fixed. There can be no insert (CAF-z0) or inserts of 8 (CAF-z8), 11 (CAF-z11), or 19 (CAF-z19) amino acids, respectively. Species is not determined and could be from e.g. human-derived (human CAF), rat derived (rat CAF), mouse-derived (mouse CAF), chicken etc. For instance, human CAF-z8 represents the 22kd C-terminal human-derived agrin fragment generated by Neurotrypsin cleavage of Agrin with an 8 amino acid insert at the Z position. |
| C44 | Artificial 44 kd C-terminal agrin fragment comprising the LG2, EGF4 and LG3 domains. Insert at the Y position can be 0, 4, 17 or 21 amino acids. Insert at the Z position can be no insert, 8, 11 or 19 amino acid sequence. Species is not determined and could be from e.g. human, rat, mouse, chicken etc. For instance, human C44-y4z8 represents the human derived 44 kd C-terminal agrin fragment comprising the LG2, EGF4 and LG3 domains having the 4 amino acid insert at the Y position and 8 amino insert at the Z position. |
| C44K/A | Artificial 44 kd C-terminal agrin fragment comprising the LG2, EGF4 and LG3 domains. The beta-cleavage site for Neurotrypsin has been deleted by replacing the lysine (K) in the cleavage site by an alanine (A). Insert at the Y position can be 0, 4, 17 or 21 amino acids. Insert at the Z position can be no insert, 8, 11 or 19 amino acid sequence. Species is not determined and could be from e.g. human, rat, mouse, chicken etc. For instance, human C44K/A-y4z8 represents the human derived 44 kd C-terminal agrin fragment comprising the LG2, EGF4 and LG3 domains having the 4 amino acid insert at the Y position and 8 amino insert at the Z position. |
| C90 | Naturally occurring 90kd agrin fragment generated by Neurotrypsin cleavage of Agrin at the alpha and beta sites. The C90 agrin fragment is located between the alpha and beta cleavage sites. The 90-kD size corresponds to the apparent running position on PAGE gel. It can have inserts at the y position. Species is not determined and could be from e.g. human, rat, mouse, chicken etc. For instance, human C90-y4 represents the human derived 90 kd agrin fragment having the 4 amino acid insert at the Y position. The z region is not present on this fragment. |
| C110 | Naturally occurring C-terminal 110kd agrin fragment generated by Neurotrypsin cleavage of Agrin at the alpha site. The 110-kD size corresponds to the apparent running position on PAGE gel. Can have inserts at the y position. Species is not determined and could be from e.g. human, rat, mouse, chicken etc. For instance, human C110-y4z8 represents the human derived C-terminal 110 kd agrin fragment having the 4 amino acid insert at the Y position and 8 amino insert at the Z position. |

### Description of different agrin fragments

In total 24, naturally occurring neurotrypsin derived-C-terminal agrin fragments are possible. These fragments may have different functions in the various organs, tissues. It is clear that distinguishing between these various agrin fragments for precise diagnosis is very important.

EP 1 990 420 by the same applicants as the present application, describes the use of the C-terminal 22-kDa agrin fragment (CAF) of agrin as biomarker for the in vivo activity of neurotrypsin and in diagnosis and monitoring of neurotrypsin-related disturbances. The applicants found out that the presence and an elevated amount of this fragment which is liberated after cleavage of agrin at the beta-site and can be measured in body fluids such as serum correlates with certain disturbances like e.g. sarcopenia.

The presence of elevated CAF-levels could also be shown in patients suffering from dysfunctions of the kidney and the lung or suffering from diseases of the brain like autism or mental retardation. Patients suffering from mild cognitive impairment, early dementia or Alzheimer can have elevated levels of CAF in cerebrospinal fluid (CSF).

Alternatively, CAF levels can be reduced compared to normal healthy persons in patients suffering from neuropathic pain. It was found that peripheral nerve injury decreased agrin expression in the ipsilateral spinal dorsal horn of rats displaying tactile allodynia. Agrin modulates neuropathic pain through NR1 phosphorylation in GABA neurons. NR1 is part of the NMDA receptor, the NMDA receptor forms a heterotetramer between two NR1 and two NR2 subunits. The NMDA receptor, a glutamate receptor, is the predominant factor in diseases where there is a modulation of the NMDAR function: like Ischemia, Seizures Parkinson disease, Huntington's disease, Pain, Diabetes (peripheral NMDAR involved), Multiple Sclerosis, Schizophrenia, Autism, Alzheimer Diseases and other dementias or cognitive impairments molecular device for controlling synaptic plasticity and memory function.

Agrin reduction, and subsequent reduction in CAF levels may also open new approaches for detection of not only neuropathic pain, but also epilepsy, tremors, and spasticity (Cui and Bazan, 2010).

As was reported CAF inhibits the alpha 3 subunit of NaK-ATPase which could be a common triggering factor in the observed dysfunctions or diseases respectively.

Summing up agrin mediates accumulation of acetylcholine receptors (AChRs) at the developing neuromuscular junction, but has also been implicated as a regulator of central nervous system (CNS) synapses (Matsumoto-Miyai, Sokolowska et al., 2009). It has been shown that the agrin C22 construct, which represents the naturally occurring neurotrypsin cleavage product, constitutes a well-folded, stable domain. Additionally the C-terminal region of agrin has been shown to bind to the alpha3 subunit of the sodium-potassium ATPase (NKA) in CNS neurons suggesting that alpha3NKA is a neuronal agrin receptor.

From the above it appears that especially CAF but also other C-terminal fragments of agrin which can be detected in different tissues and correlated to different pathologic and non-pathologic conditions is not only an important marker but also an important target, which can be used in treatment of patients.

However, promising medical applications using e.g. CAF as target require antibodies which:
allow a discrimination between human CAF (derived by specific neurotrypsin cleavage) and other human agrin fragments
are able to bind to human CAF with high specificity and affinity and
distinguish human CAF with respect to the tissue where cleavage of agrin by neurotrypsin took place.

Antibodies against various agrin fragments are available but they do not detect or only insufficiently work with human derived CAF. Monoclonal antibodies that are able to detect human CAF are not available at present.

In a Poster "Agrin serum level as a marker of sarcopenia" presented by Vrijbloed at al. on November 17, 2010 and also in EP 1990420 cited above the use of specific, affinity-purified polyclonal antibodies is described which were generated by the applicants in their laboratory against a 90-kDa and the 22-kDa fragment of agrin for detection of the 22-kDa fragment (CAF). These polyclonal antibodies G92 (Goat) and R139 (Rabbit) (Stephan et al ref) were derived from goat and rabbit and recognized non human agrin fragments under special optimized laboratory conditions, however, monoclonal antibodies with their much better specificity and reproducible properties are clearly superior over polyclonal antibodies for diagnostic applications For instance, Polyclonal antibodies can not be produced in large scale and in consistent quality. Thus it can be doubted that they would work in standard applications and they clearly did not fulfill the above requirements.

The object of the invention is to provide improved antibodies against human CAF and human CAF including agrin fragments.

### SUMMARY OF THE INVENTION:

The applicants have found out that such improved antibodies can be obtained from hybridoma cell lines which have been produced by a method according to claim 1 in which a special C44-fragment of agrin, namely a fragment C44y≥4 is used for immunization.

The term C44-fragment shall encompass all different variants of the C-terminal portion of human agrin spanning from leucine 1637 to proline 2045, with the exact position of the proline depending on the length and presence of inserts in the y- and/or z position.

The term C44y≥4-fragment shall encompass all different variants of C44-fragments having an y-insert of at least 4 amino acids. As a rule naturally occuring C44y4, 17, 21 inserts can be used. However, also synthetic fragments having different inserts are conceivable. The suffix K/A when used in this application shall mean that the agrin-fragment in question is neurotrypsin resistant.

It has surprisingly turned out that C44-fragments having an y-insert of at least 4 amino acids show a higher immunological effect compared to other C44-fragments lacking this insert. According to the observations of the applicants fragments having an y-insert tend to form aggregates which is not the case with other C44-fragments lacking this insert and which probably is the reason for the improved immunological effect.

A C44y≥4-fragment especially preferred for immunization is the fragment C44K/A-y4z8 described in detail later on. It has turned out that immunization by using this fragment leads to hybridoma cell lines which produce very specific and effective antibodies.

### BRIEF DESCRIPTION OF THE DRAWINGS.

- **Fig. 1:**: is a schematic representation of the C44K/A-y4z8 fragment;
- **Fig. 2:**: shows the sequence of the C44K/A-y4z8 fragment, with the binding sites of the antibodies represented in bold italic letters and the portions of the sequence in which epitope sequences are located depicted several times if necessary (one time for each antibody);
- **Fig. 3:**: is an ELISA showing the immunogenicity of different agrin variants in mice sera;
- **Fig. 4:**: pepspot membranes obtained for each antibody tested after epitope mapping and corresponding schemes;
- **Fig. 5:**: Western Blot showing the ability of different antibodies to detect CAF spiked in rat serum;
- **Fig. 6:**: is a Western blot showing staining of CAF-z8 and CAF-z0 with antibody 13E8;
- **Fig. 7:**: is a dot blot showing the different binding of antibodies 28H7G3 and 13E8 to CAF-z8 and CAF-z0;
- **Fig. 8:**: shows the result of an ELISA test of the antibodies 14C5G4 and 12A11D11;
- **Fig. 9:**: is a Western blot showing the binding of antibodies 2D7D9 and 28H7G3 to CAF-z0 and CAF-z8;
- Fig. 10:: shows the result of an ELISA test of z8 specific antibodies.

### DETAILED DESCRIPTION OF THE INVENTION:

The C44K/A-y4z8-fragment of agrin preferably used in the method according to the invention is schematically illustrated in Fig. 1. It can be obtained as described in Example 1.

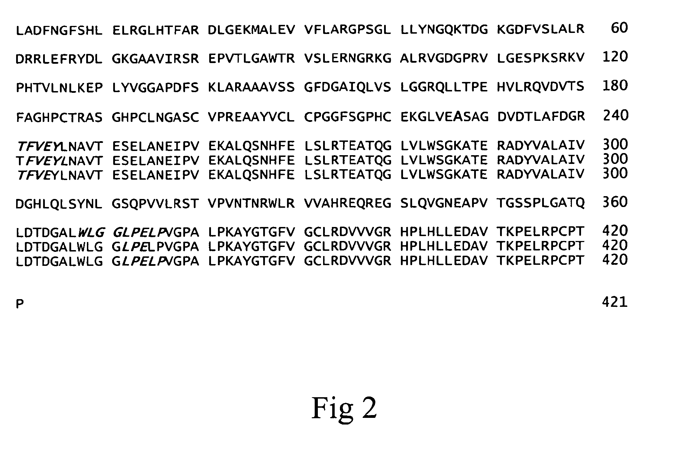

Its sequence (SEQ ID No.1) is shown in Fig. 2 with the binding sites of the antibodies obtained according to the invention being represented in bold italic letters.

The C44K/A-y4z8 fragment spans from leucine 1637 to proline 2045 of human agrin (Uniprot 000468-1 but with the y4-insertion KSRK (SEQ ID No. 2) between proline 1751 and valine 1752 and the z-insertion ELANEIPV (SEQ ID No. 3) between serine 1884 and glutamate 1885) and includes the LG-domains LG2 and LG3. The beta-cleavage site for Neurotrypsin has been deleted by replacing the lysine (K) in the cleavage site by an alanine (A: represented in bold in Fig.1). However, the invention is not limited to this special C44 fragment, but shall encompass all different variants of C44y-fragments. It is also conceivable to use the naturally occurring C44y17-fragment with the y17-insertion having the sequence VLSASHPLTVSGASTPR (SEQ ID NO: 4), or the C44y21-fragment with the y21-insertion having the sequence KSRKVLSASHPLTVSGASTPR (SEQ ID NO: 5). The same is applicable to the z-insertion. Apart from the preferably used C44-fragment with a z-8 insertion it is also possible to use C44-fragments having no z-insertion, a z11-insertion of the sequence PETLDSGALHS PETLDSGALHS (SEQ ID NO: 6) or a z19-insertion of the sequence ELANEIPVPETLDSGALHS (SEQ ID NO: 7).

The applicants have prepared an antibody-platform containing a number of different hybridoma cell lines producing monoclonal antibodies named 28H7G3, 264E12B8, 28A6H11, 14C5G4, 12A11D11, 28F7A6, 14B7B8, 264B12A8 and 13E8 by using the method according to the invention. The method is discussed in detail in Examples 2 and 3.

Apart from the hybridoma cell line, producing antibody 13E8 (described below), all hybridoma cell lines have been deposited by the applicants under the Budapest Treaty on January 11, 2011 at DSMZ - Deutsche Sammlung von Mikroorganismen und Zellkulturen GmbH, Inhoffenstraße 7 B, 38124 Braunschweig GERMANY. The cell lines were accorded the accession numbers:

| | |
|---|---|
| 28H7G3 = DSMACC3101; | 264E12B8 = DSMACC3102 |
| 28A6H11 = DSMACC3103; | 14C5G4 = DSMACC3104 |
| 12A11D11 = DSMACC3105; | 28F7A6 = DSMACC3106 |
| 14B7B8 = DSMACC3107; | 264B12A8 = DSMACC3108 |

In Fig. 1 the binding sites of the antibodies on the depicted C44K/A-y4z8-fragment of agrin are schematically shown. The epitope sequences of the antibodies were determined by peptide mapping (Example 5) and are as following:
Antibody 28H7G3 (SEQ ID No. 8) Sequence:TFVEY
Antibody 14B7B8 (SEQ ID No. 9) Sequence: FVEYL
Antibody 28A6H11 (SEQ ID No. 10) Sequence: TFVE
Antibody 264E12B8 (SEQ ID No. 11) Sequence: WLGGLPELP
Antibody 264B12A8 Sequence: LPE
Antibody 28F7A6 (SEQ ID No. 12) Sequence: LPELP

As it appears the epitopes mentioned above have not been described so far. It is conceivable that also the knowledge about the epitopes can lead to favorable applications. E.g. it could be conceivable to create synthetic peptides including one or more of the eptiope-sequences which could be used in therapy or prophylactic applications. Furthermore the invention is not limited to the specific antibodies mentioned above but shall also encompass other antibodies which bind to the epitopes in question.

Also antibodies 14C5G4 and 12A11D11 and the antibody 13E8 mentioned above, were obtained using the method according to the invention. For these antibodies no epitope sequences were determined.

However, it could be shown (Example 7) that antibody 13E8 binds specifically to CAF z-8 fragments indicating that its epitope-sequence must be ELANEIPV (SEQ ID No. 3) or a portion thereof. For a person skilled in the art it is evident that by using the method according to the invention also further antibodies not described in this application can be obtained which bind specifically to other inserts possibly present in the C44-fragment, like the y4, y17 or y21 or the z11 or z19 inserts.

Antibodies 14C5G4 and 12A11D11 were shown to bind to the non-CAF N-terminal portion of C44 (Example 8).

Fig. 1 sums up the different binding sites of the above antibodies. As one can see in Fig. 1 antibodies 28H7G3, 14B7B8, 28A6H11, 264E12B8, 264B12A8, 28F7A6 and 13E8 bind to sites located in the CAF-portion with antibody 13E8 binding specifically to a z8-insert possibly present in the CAF-fragment. Antibodies 14C5G4 and 12A11D11 bind to sites located in the other N-terminal portion of C44.

Consequently by using the antibodies obtained by the invention it is possible to discriminate CAF-fragments from other fragments and furthermore as will be discussed later it is also possible to determine the origin of a CAF-fragment detected.

By using e.g. antibody 28H7G3 and antibody 14C5G4 in a test system it is possible to determine whether or not the agrin fragment detected in a sample is the CAF-fragment or a larger fragment including the CAF-portion, e. g. the 110 kDa-fragment which is generated by cleavage of agrin at its alpha cleavage-site. If both antibodies bind to the fragment, this will suggest a larger agrin-fragment including the CAF-fragment; if only one of the antibodies binds this will suggest either CAF (28H7G3) or an agrin fragment lacking CAF (14C5G4).

Interestingly, 14B7B8 recognizes only CAF and no longer C-terminal agrin fragments, for instance human C44 or human C110 whereas 28H7G3 detects all CAF containing variants to human CAF, C44 and C110. Although both antibodies appear to bind to the same epitope, the antibodies have different clearly different binding characteristics. The use of 14B7B8 allows for specific detection of human CAF without interference of other agrin fragments whereas 28H7G3 can simultaneously detect all CAF containing fragments.

The use of a specific combination of the mentioned antibodies obtained according to the invention will allow for the establishment of ELISA systems discriminating the various C-terminal agrin fragments with respect to concentration and identity.

With regard to antibody 13E8 as stated above this antibody binds specifically to a z8-insert possibly present in the CAF fragment. As this z8-insert is only present in CAF fragments originating from neural tissues, the use of this antibody will allow a decision whether or not the fragment detected has been liberated in neural tissue or in other tissues.

It is clear that antibodies obtained by the method according to the invention allow a number of different applications in diagnosis but also in the treatment of CAF related disturbances.

As stated above the CAF fragment could be a toxic protein fragment as it inhibits the alpha 3-unit of Na,K,-ATPase. This could lead to intra, or extracellular toxic sodium, potassium or calcium concentrations. So one important application of the antibodies generated according to the method of the invention could be to use them for clearing the blood of patients suffering from kidney-diseases in an artificial kidney. This clearing can be achieved by attaching the antibodies to an insoluble carrier material such as Sepharose (cross-linked dextrane) or other biocompatible materials and exposing the patient's blood to the antibodies in an apheresis procedure similar as this has been described for the removal of other proteins such as low density lipoproteins (LDL) (Borberg, Gaczkowski et al., 1990). Preferably the blood is anticoagulated and freed from cells before contact with the antibody.The bound antibody can be regenerated by exposure to concentrated salt solutions and/or low pH-values.

It is also conceivable to manufacture a medicament using one or more of the antibodies which can be obtained according to the method of the invention for a direct neutralization of CAF in a patient by intravenous or intradermal injection or infusion.

There is a number of further applications conceivable so that one main aspect of the invention is directed to the use of the antibodies in pharmaceutical formulations or the use of these antibodies in the manufacturing of such formulations. This may also include a humanization procedure of the antibody in order to prevent allergic reactions or its neutralization by antibody formation.

Such formulations can be directed against elevated CAF levels in blood and can be used in for instance sarcopenia, kidney diseases, lung diseases, and diseases characterized by mental retardation like Alzheimer's disease, Parkinson disease .

It is also conceivable to use the antibodies for the detection of agrin or agrin fragments as marker or surrogate marker in clinical trials or as marker in personalized medicine. In clinical trials with CAF containing drugs the mABs can be used to detect the amount of drug in the body, for instance in blood or urine.

One such drug is e.g. a neurotrypsin-resistent C44-fragment of agrin (as described in patent application EP 09011367.1). The mABs can be used for dermination of the concentration of this neurotrypsin-resistant agrin fragment in patients during clinical trials or in any other patients that the takes this fragment.

The following examples illustrate the invention, but are not limiting it. The skilled person in the field reading these examples will be able to apply other related conditions and these are also within the scope of the invention.

### EXAMPLES:

### Example 1: Cloning, expression and purification of C44K/A-y4z8

### a) Cloning of neurotrypsin-resistant human 44-kd C-terminal fragment of agrin

Initially, full length human agrin y0z0 but lacking the N-terminal NtA domain (Human agrin y0z0 deltaNTA starts at position K156 in the protein sequence of accession number NP_940978) was cloned by PCR into the pEAK8 vector containing the coding sequence for the secretion signal of human calsyntenin-1, (Reif, Sales et al., 2007) via appropriate restriction sites and primers. As template for human agrin the vector pCMV-XL5-Agrin (purchased from Origene USA) was used.

In two subsequent steps, the corresponding codons required for the y4z8 insertions were introduced by site directed mutagenesis using standard techniques resulting in the pEAK8 vector containing full length human agrin y4z8 deltaNtA.

Using this vector as template, the gene coding for the 44-kd C-terminal fragment of human agrin was amplified introducing the coding region for a His8 tag and a prescission protease cleavage site at the N-terminus of the translated protein.

The neurotrypsin-resistant form of human Agrin C44K/A was generated in a quick change mutagenesis step using primers which introduce the codon for an alanine at the place of the codon for the lysine in the cleavage site- □ of agrin.

This plasmid generates a protein with the sequence of SEQ ID NO: 1 in the culture supernatant of transfected cells (without signal sequence) with the additional N-terminal amino acid sequence "ARVNHHHHHHHHLEVLFQGP" (SEQ ID No. 13) containing the His8 tag and the prescission cleavage site.

### b) Expression and purification of human agrin C44 and neurotrypsin-resistant C44K/A

500 ml HEK 293 cells grown in Excell 293 medium to a density of 1 x 10⁶ cells/ml were pelleted by centrifugation in a Sorvall RC5C centrifunge at 100 x g for 30 min. The cells were resuspended in 500 ml RPMI1640 medium prewarmed to 37 °C. 1.25 mg of pEAK8 containing the insert for the expression of neurotrypsin-resistant human AgrinC44 y4z8 were diluted in 25 ml 150 mM NaCl. 3.75 mg polyethylene imine (PEI), 25 kDa, were diluted in 25 ml 150 mM NaCl. Both solutions were pooled and incubated at room temperature for 10 min. Afterwards this solution was added to the cell suspension which was transferred to a 1000 ml spinner flask. The cell suspension was incubated for 7 days at 75 rpm on a stirring platform placed in an incubator with 5 % CO₂ and 37°C in humidified atmosphere.

After 7 days, the culture supernatant was harvested by centrifugation at 5000 rpm in a Sorvall RC5C centrifuge. Remaining particles were removed by filtration through a 0.22 µm Millipore sterile filtration device. The filtrated culture supernatant was concentrated 10 times using a Pellicon PLCGC 10 kDa cuttoff tangential flow cartridge and dialyzed at least 1:1000 against 20 mM MOPS pH 8.5, 400 mM NaCl. The dialysate was subjected to immobilized metal affinity chromatography (IMAC) taking advantage of the His8 tag using a 10 ml bench top His Select column labeled with Ni²⁺. After loading of the concentrated and dialyzed cell culture supernatant, the column was washed with 100 ml dialysis buffer and bound protein was eluted with 5 times 10 ml of dialysis buffer containing 500 mM imidazole. The purification success was followed by SDS-PAGE. Positive fractions were pooled and concentrated 10 times with an AMICON 30 kDa cuttoff filtration device at 3000 x g in a Sigma 4K15 centrifuge and dialysed 1:10000 against 20 mM MOPS pH 8.5, 200 mM NaCl. The concentration of the purified C44K/A was determined via UV spectroscopy using the extinction coefficient of 0.725 cm²/mg.

### c) Removal of the His8 tag by Prescission protease cleavage

In case the His8 tag should be removed, the buffer of 0.5 ml protein solution was exchanged to 50 mM Tris-HCl, pH 7.2, 150 mM NaCl, 1 mM DTT, 1 mM Na₂EDTA using a NAP-5 column pre-equilibrated with that buffer. The protein solution was eluted in 1 ml of the same buffer. 1 µl 1M DTT was added to the protein solution and mixed by flipping the tube several times. 20 µl of Prescission protease (1U/ul) was added and the tube was mixed by flipping it several times. The reaction was done overnight at 4 °C. A 0.5 ml gravity flow glutathione sepharose column was equilibrated with 5 ml PBS supplemented with 1 mM DTT. The digested protein solution was loaded and the flow through collected. The column was washed with 3 times 1 ml PBS supplemented with 1 mM DTT and the flow through was collected in the same tube as the previous flow through fraction. The collected flow-through fractions were dialysed 2 times 2 hours against 5 l of 20 mM MOPS pH 8.5, 400 mM NaCl to remove DTT and EDTA.

To remove the cleaved His8 tag a second IMAC was performed. A 1 ml Chelating sepharose FF column previously labelled with Ni²⁺ ions was equilibrated with 5 ml 20 mM MOPS pH 8.5, 400 mM NaCl. The dialysed protein solution was applied onto the column and the flow through collected. The column was washed with 3 times with 1 ml 20 mM MOPS pH 8.5, 400 mM NaCl and the flow though collected in the same tube. The pooled fractions were concentrated with a AMICON 30 kDa cutoff concentrator to 0.5 ml. and the buffer was exchanged using a NAP-5 column pre-equilibrated with 20 mM MOPS pH 8.5, 200 mM NaCl. The concentration was determined via UV spectroscopy using the extinction coefficient of 0.761 cm²/mg. The protein was freshly used for further experiments or stored at -80 °C until usage. The protein sequence of the corresponding protein corresponds to the portion of SEQ ID NO: 1 with the additional N-terminal amino acid sequence "GP" as a result of the prescission cleavage.

### EXAMPLE 2: Immunization of mice with C44K/A-y4z8

Three 6-8 weeks old female Balb/c mice were immunized with 90 - 150 microgram of C44K/A in complete Freund's adjuvans. After 28 days, C44K/A was administered in incomplete Freund's adjuvance. This step was repeated after 56 days. After 87 days, C44K/A was administered in PBS, which was repeated at day 90. One day later, cells from the knee lymph knots were prepared and the resulting B-cells are fused with P3-X63-Ag8 mouse myeloma cells in the presence of PEG4000.

After completion of the cell fusion the cells were plated on 24-well plates which results in 360 oligo-clones consisting of 5-10 clones. Cells were cultivated for 10 days in selective Optimem medium (GIBCO) selecting for fused cells (hybridoma) only.

Supernatants were screened by ELISA for positivity. Single clones were generated by a two fold limited dilution. Positivity of the clones was checked by ELISA with C44K/A.

### EXAMPLE 3: Expression and purification of monoclonal antibodies

Hybridoma cells were adapted to serum free ISF-1 medium (BIOCHROME AG) and grown for 5-7 days. Approximately 100 ml conditioned medium was subjected to protein-G sepharose chromatography.

In brief, the conditioned supernatant was loaded on a 1 ml protein-G or protein-A sepharose column (GE-HEALTHCARE) with a flow rate of 1.5 ml/min. After washing with 30 column volumes (CV) with PBS/0.5M glycine pH 7.4 the bound antibody is eluted with 100 mM citrate buffer pH 2.6. Positive fractions are pooled and neutralized by an appropriate amount of 2M Tris.

The purified antibody is dialysed against PBS and stored at 4 °C until further use of it.

### EXAMPLE 4: Immunisation with CAF-z8 and C44K/A-y4z8 and comparison of the antibodies obtained with these immunogens

For each antigen, C44K/A-y4z8 or CAF-z8, 3 mice were used. 5 mg/kg of the corresponding agrin variant was injected subcutaneously for 24 days once a day. After this, the mice were sacrificed and the serum was prepared using standard serum tubes

### Coating of ELISA plates

Nunc MaxiSorp Immuno plates 96 well (flat bottom, polystyrene) were coated with 100 ng/well of CAF-z8. Coating was done over night (i.e. for 15 h) at 4°C in presence of 1x Candor Coating Buffer, pH 9.6.

After aspiration of the coating buffer the plates were washed 7 times with 400 µl Na-PBS-CAS, pH 7.2 (PBS supplemented with additional 0.2 mol NaCl ("Na") and with 0.05% casein ("CAS")). Washing was done using a BioTek ELx405 plate washer (Witec AG). The wells of the washed plates were blocked by adding 180 µl of Candor Blocking Solution per well. The plates were subsequently incubated for 3 h at room temperature. After removal of the Blocking Solution the plates were washed 7 times with 400 µl Na-PBS-CAS, pH 7.2. Subsequently, the plates were used for an ELISA experiment.

### Checking immunogenicity of agrin variants

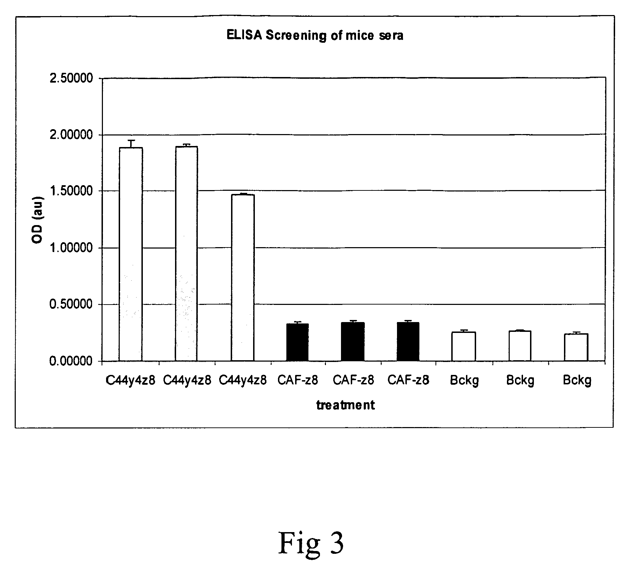

To PBS casein and Tween 20 were added to final concentrations of 0.05% (PBST-CAS buffer). Sera were 1000 fold diluted with PBS. A background control was prepared from untreated mice. Of each sample 100 µl per well were transferred to the ELISA plates coated with the corresponding agrin species. After incubation of the plates for 2 h at room temperature (RT: 20-21 °C; sealed with a sealing film) they were washed 7x with 400 µl PBST-CAS using a BioTek ELx405 plate washer (Witec AG). Subsequently, 100 ul/well of Goat-anti-Mouse, Peroxidase Labeled; KPL, 074-1806) were added, diluted 10'000x with PBST-CAS. The plates were afterwards incubated for 30 min at RT and then washed 7x with 400 µl PBST-CAS. Substrate was added (100 µl per well; "TMB Super Sensitive One Component HRP Microwell" substrate; BioFX; TMBS-1000-01) and the plates were incubated for 30 min at RT. The color development was stopped by adding 100 µl of 450 nm Stop Reagent (BioFX; # STPR-1000-01) to each well. Subsequently, the absorbance at 450 nm was measured using a Tecan infinite F200 plate reader. The results of the measurement are summarized in Fig. 3. No good immune response was detected in sera from CAF-z8 mice. The response was just detectable above background (bckg) with an average of 0.07 OD units. Surprisingly a very high immune response was seen in mice treated with C44K/A-y4z8 as antigen. The average increase above background (bckg) was 1.6 OD units. The reason for the absence of a good immune response of CAF-z8 is not completely understood but it has been shown that the agrin C22 construct, which represents the naturally occurring neurotrypsin cleavage product, constitutes a well-folded, stable domain (tydor). In contrast the C44K/Ay4z8 is a protein that is prone to aggregate (data not shown). This aggregation could be the reason to the strong immune response in mice. The lack of a good immune response of CAF in mice might be one of the reasons that good monoclonal antibodies against human CAF are not commercially available as yet. However using C44K/A-y4z8 as antigen a strong immune response was observed which resulted in large variety of monoclonal antibodies.

### EXAMPLE 5: Epitope mapping of anti-CAF antibodies

For the mapping of the epitopes of the antibodies against CAF a Pepspot membrane from JPT Peptide Technologies, Berlin, Germany was used: the sequence of the LG3 domain, starting with the P1' residue of the neurotrypsin cleavage site beta of human agrin (CAF-z0) was split into peptides of 15 amino acids length with an overlap of 14 amino acids. This resulted in 172 peptides of 15 amino acids length covering the whole sequence. The peptides were N-acetylated and covalently linked via their C-termini to a cellulose-□ □alanine membrane.

The membrane was incubated with antibodies at a concentration of 0.1 - 2 microgram/ml in PBS supplemented with 0.1 % Tween 20 (PBST) for 2 hours. After 3 washing steps for 10 min with PBST, a goat-anti-mouse secondary antibody labelled with HRP was added and incubated for 30 min. After 3 times washing with PBST for 10 min, Immobilon Western chemiluminescent HRP substrate (MILLIPORE) was added and the membrane is recorded on a Stella imaging system (RAYTEST) for 1 min. Positive spots were assigned to the corresponding peptide spots and the epitope was matched.

The positive spots are assigned to the peptide sequences spotted onto the membrane. The essential amino acids contributing to the binding of the antibodies were located by determination of the first amino acid giving a signal at the N-terminal side and the last amino acid giving a signal at the C-terminal side of the protein sequence.

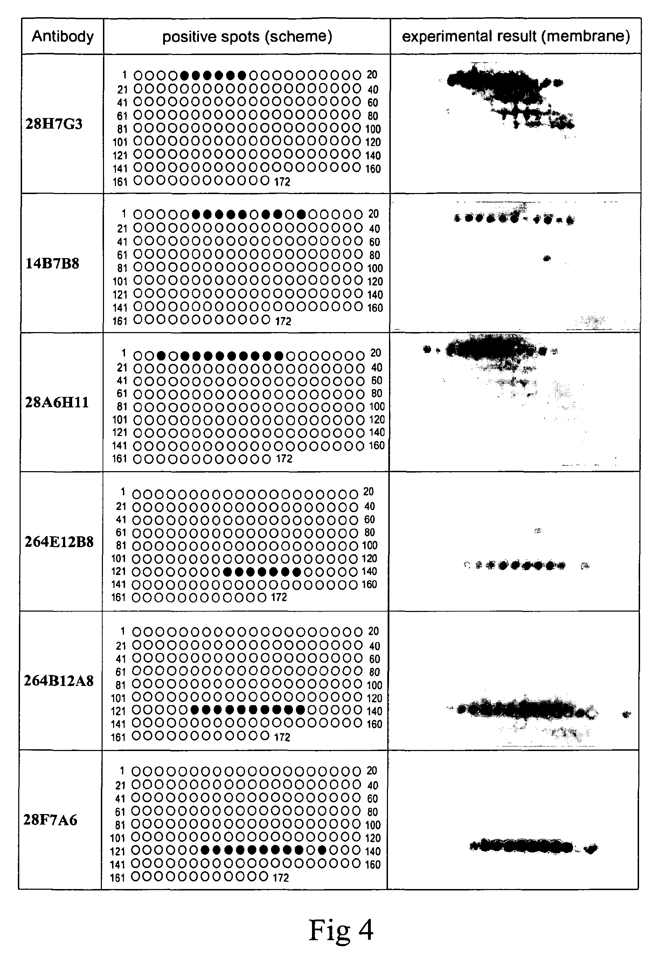

The results of the peptide mapping are represented in Fig. 4 and result in the following epitopes shown in Table 2.

**Table 2**

| Antibody | Epitope-sequence |
|---|---|
| 28H7G3 | TFVEY (SEQ ID No.8) |
| 14B7B8 | FVEYL (SEQ ID No. 9) |
| 28A6H11 | TFVE (SEQ ID No. 10) |
| 264E12B8 | WLGGLPELP (SEQ ID No. 11) |
| 264B12A8 | LPE |
| 28F7A6 | LPELP (SEQ ID No. 12) |

### Epitope sequences of antibodies obtained according to Example 1

### EXAMPLE 6: Western Blotting of CAF spiked in rat serum

To demonstrate the ability of the antibodies to detect CAF in biological samples, human CAF-z0 was spiked into rat serum, which was diluted 1:250 with PBS, to a concentration of 0.2 ng/ul. 10 µl corresponding to 2 ng CAF-z0 were loaded on a 4-12% bis-tris SDS-PAGE gel (BIORAD) and blotted on a PVDF membrane (MILLIPORE) using a semi dry blotting apparatus (BIORAD).

The blot was incubated with 1 ug/ml of the monoclonal antibodies in 10 % roche blocking reagent in PBST o/N. For a comparison a commercially available antibody against CAF, ab247 from ABCAM was used.

After washing and incubation with HRP-conjugated goat-anti-mouse antibody, the chemiluminescent signals derived from the HRP reaction with chemiluminescent substrate (MILLIPORE) was recorded for 1 min in a Stella imaging system (RAYTEST).

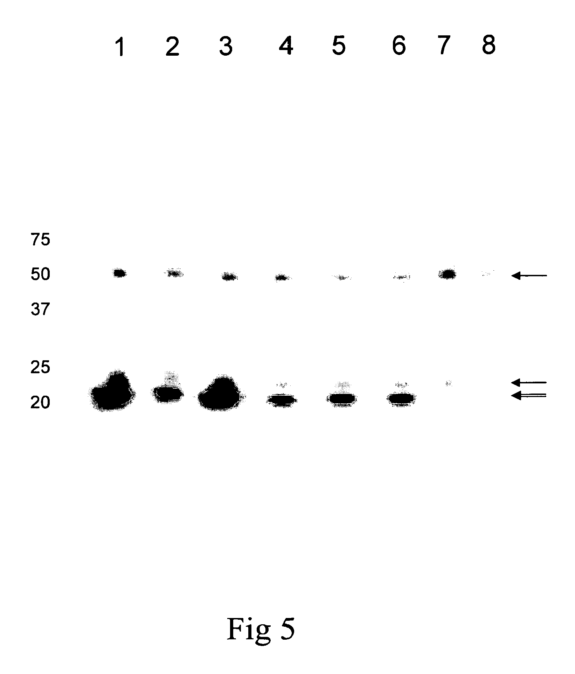

The results are shown in Fig. 5. Lane 1 = Antibody 28H7G3; Lane 2 = Antibody 14B7B8; Lane 3 = Antibody 28A6H11; Lane 4 = 264E12B8; Lane 5 = Antibody 264B12A8; Lane 6 = 28F7A6; 7 = Abcam antibody, [Agr 247] ab12364-100; lot:577087 and Lane 8 = HRP-conjugated goat-anti-mouse IgG antibody, Socochim SA, 074-1806

Numbers and explanation correspond to the used primary antibodies. Note the bands of the heavy and light chains of the antibodies naturally present in rat serum (indicated by single line arrows at 55 kDa and 25 kDa, respectively) which are detected by the secondary anti-mouse antibody. These bands serve as a loading control and as a functionality control of the Western blot.

CAF signals indicated by the double line arrow at 22 kDa could only be detected in Lanes 1-6, i.e for the antibodies obtained according to the invention.

### EXAMPLE 7: Monoclonal antibody 13E8 specific for the z-splice site

The agrin CAF-z8 specific antibody 13E8 was derived from the same immunization described in Example 1 but without limiting dilution. For the selection of this clone the following procedure was chosen:
Coating of ELISA plates: Nunc MaxiSorp Immuno plates 96 well (flat bottom, polystyrene) were coated with 100 ng/well (125 ul/well) of human CAF-z8 or human CAF-z0. Coating was done over night (i.e. for 15 h) at 4°C in presence of 1x Candor Coating Buffer, pH 9.6.

After aspiration of the coating buffer the plates were washed 7 times with 400 µl Na-PBS-CAS, pH 7.2 (PBS supplemented with additional 0.2 mol NaCl and with 0.05% casein). Washing was done using a BioTek ELx405 plate washer (Witec AG). The wells of the washed plates were blocked by adding 180 µl of Candor Blocking Solution per well for 3 h at room temperature. After removal of the Blocking Solution the plates were washed 7 times with 400 µl Na-PBS-CAS, pH 7.2. Subsequently, the plates were used for an ELSA experiment.

For checking putative anti-agrin antibody containing hybridoma supernatants wash buffer (PBS supplemented with 0.05% Casein and Tween 20) was freshly prepared. Cell culture supernatants were 30-fold diluted with PBS. A negative control was prepared with cell culture medium only. Of each sample 100 µl were transferred to a well of the ELISA plates coated with the various agrin species.

After incubation of the plates for 2 h at room temperature (RT: 20-21°C; sealed with a sealing film) they were washed 7x with 400 µl PBST-CAS using a BioTek ELx405 plate washer (Witec AG). Subsequently, 100 ul/well of 10000 fold diluted Goat-anti-Mouse, Peroxidase Labeled (KPL, 074-1806) were added and the plates were afterwards incubated for 30 min at RT.

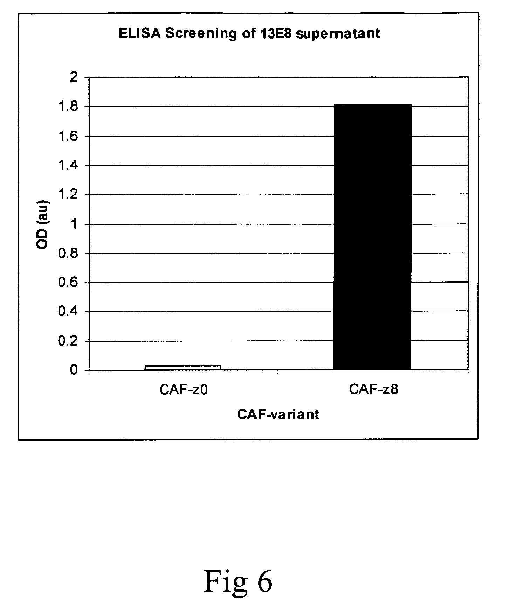

The plates were washed 7x with 400 µl PBST-CAS, and substrate was added (100 µl per well; "TMB Super Sensitive One Component HRP Microwell" substrate; BioFX; TMBS-1000-01). After addition of the TMB substrate the plates were incubated for 30 min at RT. The color development was stopped by adding 100 µl of 450 nm Stop Reagent (BioFX; STPR-1000-01) to each well. Subsequently, the absorbance at 450 nm was measured using a Tecan infinite F200 plate reader. The results of the measurement with clone 13E8 are summarized in Fig. 6. It is apparent that 13E8 binds with high specificity only to CAF-z8 and not to CAF-z0, which means that this antibody is specific for the z-insert in CAF.

In a further experiment the antibodies 13E8 and 28H7G3 were compared with respect to their binding of CAF-z0 and CAF-z-8. 2 ng of CAF-z0 and CAF-z8 were loaded onto a 4-12% Bis-Tris SDS-PAGE gel (BIORAD). After separation, the proteins were blotted onto a PVDF membrane (MILLIPORE) by semi dry blotting with standard transfer buffer using a Trans blot SD cell (BIORAD) for 60 min at 24 V.

For detection of the blotted proteins the purified antibodies (13E8 and 28H7G3) in a concentration of 1 ug/ml or cell culture supernatant (1:2 diluted) were used in PBST.

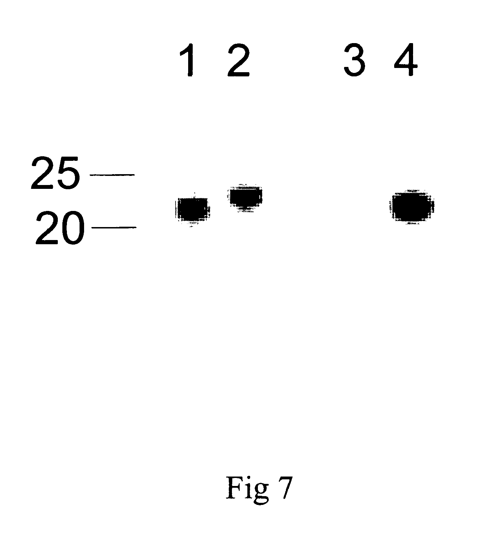

The blots were blocked for 1 h with Roche blocking solution (10%) in PBST. Afterwards, the required amount of antibody was added and incubated for 2 h. After 3 times wash with PBST the secondary antibody solution (standard goat anti mouse-HRP conjugate) was added in appropriate concentration in PBST+ 10% Roche blocking solution. After incubation for 30 min the blots were washed 3 times with PBST and the blots were exposed with chemiluminescent substrate using a Stella chemiluminescence imager. The result is shown in Fig. 7 with the lanes 1 and 2 showing the results for antibody 28H7G3 (Lane 1: CAF-z0; Lane 2: CAF-z8) and the lanes 2 and 3 showing the results for 13E8 (Lane 3: CAF-z0; Lane 4: CAF-z8).

Also these results show that the antibody 13E8 recognizes specifically CAF-z8 while 28H7G3 recognises both variants.

### EXAMPLE 8: Antibodies against the N-terminal (non-CAF) part of C44

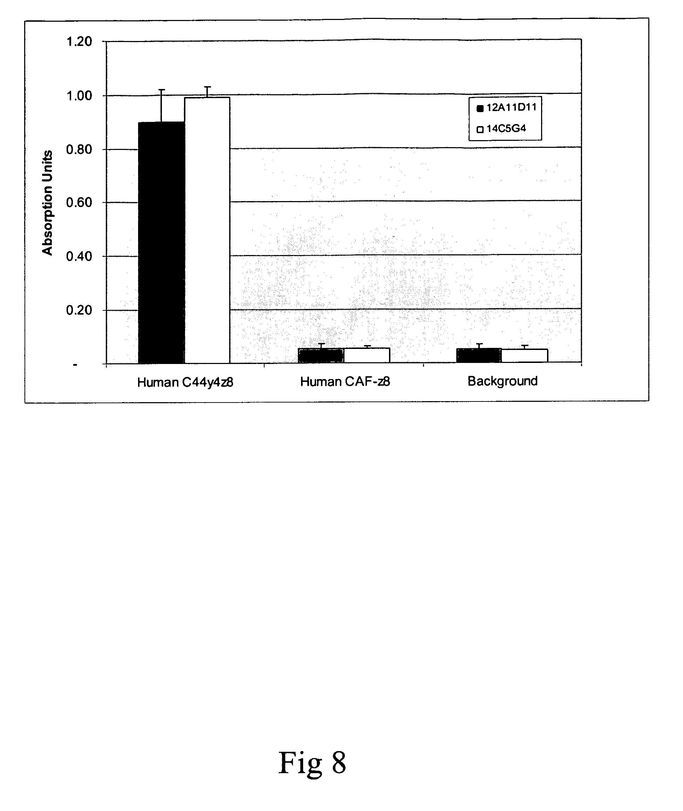

To test for antibodies raised against the N-terminal part of C44y4z8 an ELISA test was done as described in EXAMPLE 6 using C44y4z8 and CAF-z8 as proteins for coating the ELISA plates. The 2 antibodies mentioned above, 14C5G4 and 12A11D11 were tested. The result is shown in Fig. 8. Both antibodies tested bind to C44K/A-y4z8 but not to CAF-z8, which means that they bind to the N-terminal non-CAF-portion of C44.

### EXAMPLE 9: Immunizing mice to achieve a z8 splice site specific antibody

Neuronal agrin can have different biological functions as non-neuronal agrin (Bezakova and Ruegg, 2003). For instance, neuronal-Agrin mediates accumulation of acetylcholine receptors (AChRs) at the developing neuromuscular junctions whereas non-neuronal agrin does not posses this activity. The presence of CAF-z8 in blood, serum, urine or cerebrospinal fluid is indicative for degradation of neuronal agrin. A monoclonal antibody that is able to specifically detect CAF-z8 and thus can differentiate between neuronal and non-neuronal agrin is therefore very important. In order to obtain such an antibody the peptide NH2-ELANEIPV(SEQ ID No.: 3)-COOH containing the amino acids present in the z8 insertion of human agrin was conjugated to mouse ovalbumin to serve as immunogen. Amino acid synthesis, conjugation, immunization and cell fusion and monoclonal antibody purification were done according to standard techniques know to the expert. Briefly, the peptide conjugated to ovalbumin was used as antigen to immunize 4 BALB/c mice. Injections of 20-30 □g + CFA (Complete Freund Adjuvant) are done at T0 (start day), while 10-15 □g + IFA (Incomplete FA) are done at T21, T28 (days) subcutaneously. Further injections follow at T42, 47, 59, 61, 72. At day T75 splenectomy was done followed by fusion of spleenocytes with myeloma cells and screening of mother hybridoma clones by ELISA testing against the peptide coupled to KLH (Keyhole Limpet Hemocyanin). Positive clones were subcloned by limiting dilution and adapted to serum free conditions in ISF-1 medium. Antibodies secreted into the culture supernatant were purified by Protein G sepharose chromatography. The best monoclonal antibody (mAB) was an IgM (2D7D9) subclone and no IgG clones were obtained.

### Western blot and ELISA results.

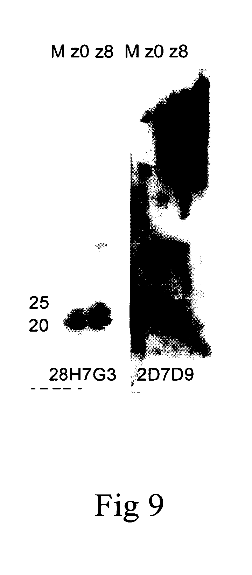

A Western blot with 2D7D9 using 28H7G3 as control antibody was performed with 100 ng of CAF-z0 (lane z0) and CAF-z8 (lane z8) blotted onto a PVDF membrane with semi dry blotting technique. The membrane was blocked for 1 hour in PBST supplemented with 10% Roche blocking solution. Afterwards, 13 ng/ml biotinylated 28H7G3 or 1 ug/ml biotinylated 2D7D9 were added and incubated for additional 2 hours. After 3 times washing for 10 min with PBST, Streptavidin-poly-HRP conjugate (PIERCE, 0.5 mg/ml) was added 1:30000 diluted in PBST supplemented with 10% Roche blocking solution. After 3 times washing for 10 min in PBST, the membrane was exposed to chemiliminescent substrate and imaged in a Stella imaging system. The panel with 28H7G3 was exposed for 1 sec while the panel with 2D7D9 was exposed for 300 sec. Considering the roughly 100 fold access of 2D7D9 over 28H7G3 used as first antibody and the 300 times longer exposure time to receive a signal for 2D7D9 one can conclude that 28H7G3 is at least 30.000 times more sensitive than 2D7D9. CAF-z0 and CAF-z8 appear as double bands due to a fraction of glycosylated protein (Fig.9).

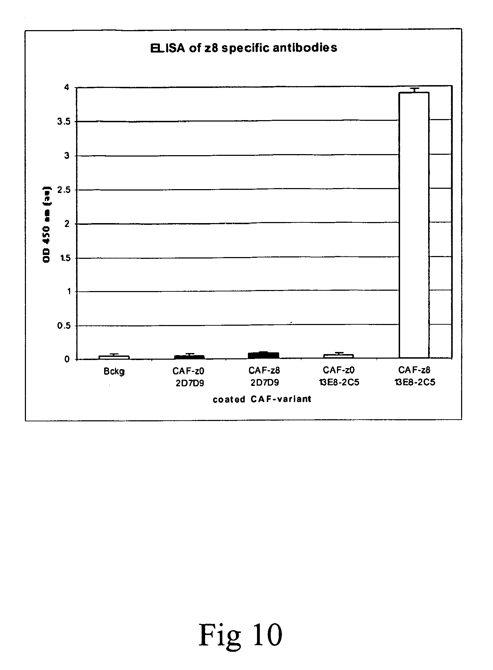

The received antibody shows only very weak signals in the Western blot without a good discrimination between CAF-z0 and CAF-z8. In ELISA, the antibody gives only weak signals with a slight discrimination between CAF-z0 and CAF-z8 (Fig. 10).

### ELISA with 2D7D9

ELISA plates (Nunc maxi-sorp immuno plate) were coated 30 ng/well CAF-z0 or CAF-z8. The plates were blocked with 180 □1 per well Candor Blocking Buffer for 4 h at room temperature. 300 ng/ml 2D7D9 or 13E8-2C5 were added and incubated for 2 h at RT. The plate is washed 7 times with 400 µl PBST supplemented with 0.05 % Tween 20 and 0.05 % casein. As detector antibody, goat anti mouse IgM-HRP resp goat anti mouse IgG-HRP were used in a dilution of 1:10000. After washing, bound antibodies are detected with TMB Super Sensitive One Component HPR Microwell substrate blocked with 100 □1 per well "450 nm Stop Reagent for TMB Microwell Substrates" from BioFX in a Tecan Infinite F200 96 well reader. The obtained signals are very weak and the discrimination of the CAF variants is poor. The immunization method was not successful in generating specific mAB with a high affinity. The mAB 13E8-1C5 (see below) obtained with immunization of C44K/A-y4z8 showed a high and specific signal for CAF-z8.

Reference List:
Bezakova G, Ruegg MA. New insights into the roles of agrin. Nat. Rev. Mol. Cell Biol., 2003; 4: 295-308.
Borberg H, Gaczkowski A, Oette K, Stoffel W. Immunosorptive apheresis of LDL. Prog. Clin. Biol. Res, 1990; 337: 163-7.
Cui JG, Bazan NG. Agrin downregulation induced by nerve injury contributes to neuropathic pain. J Neurosci., 2010; 30: 15286-97.
Matsumoto-Miyai K, Sokolowska E, Zurlinden A, Gee CE, Luscher D, Hettwer S, Wolfel J, Ladner AP, Ster J, Gerber U, Rulicke T, Kunz B, Sonderegger P. Coincident pre- and postsynaptic activation induces dendritic filopodia via neurotrypsin-dependent agrin cleavage. Cell, 2009; 136: 1161-71.
Molinari F, Rio M, Meskenaite V, Encha-Razavi F, Auge J, Bacq D, Briault S, Vekemans M, Munnich A, ttie-Bitach T, Sonderegger P, Colleaux L. Truncating neurotrypsin mutation in autosomal recessive nonsyndromic mental retardation. Science, 2002; 298: 1779-81.
Reif R, Sales S, Hettwer S, Dreier B, Gisler C, Wolfel J, Luscher D, Zurlinden A, Stephan A, Ahmed S, Baici A, Ledermann B, Kunz B, Sonderegger P. Specific cleavage of agrin by neurotrypsin, a synaptic protease linked to mental retardation. FASEB J, 2007; 21: 3468-78.
Stephan A, Mateos JM, Kozlov SV, Cinelli P, Kistler AD, Hettwer S, Rulicke T, Streit P, Kunz B, Sonderegger P. Neurotrypsin cleaves agrin locally at the synapse. FASEB J, 2008; 22: 1861-73.

## Claims

1. A method for the production of a hybridoma cell lines producing monoclonal antibodies capable to specifically binding to a human C44-fragment of agrin, comprising administering to wild-type-mice an immunizing amount of C44y≥4-fragment of agrin, isolating antibody producing cells from the immunized mice, fusing them with a myeloma cell line, growing the fused cells in a selection medium, screening the antibodies in the supernatants of hybridoma cells for binding to C44-fragment of agrin and isolating the hybridoma cells producing the desired monoclonal antibodies.

2. The method according to claim 1, wherein the fragment used for immunization is C44-y4.

3. The method according to claim 2, wherein the fragment used for immunization is C44K/Ay4.

4. The method according to claim 3, wherein the fragment used for immunization is C44K/Ay4z8.

5. A hybridoma cell line obtainable by the method according to one of the claims 1-4.

6. A hybridoma cell line according to claim 5 deposited under DSMACC3101, DSMACC3102, DSMACC3103, DSMACC3104, DSMACC3105, DSMACC3106, DSMACC3107 or DSMACC3108.

7. A method for the production of an antibody capable to specifically binding to the C44-fragment of agrin, comprising culturing a hybridoma cell line according to claim 5 and isolating the monoclonal antibody from the supernatant.

8. A monoclonal antibody obtainable by the method according to claim 7.

9. A monoclonal antibody according to claim 8, capable of recognizing the CAF-portion of agrin.

10. A monoclonal antibody according to claim 8, capable of recognizing an insert present in the C44-fragment.

11. A monoclonal antibody according to claim 10, capable of recognizing a z8- insert present in CAF-portion.

12. A method for clearing biological material from CAF comprising treating said material in vitro with a monoclonal antibody according to claim 8 and removing possibly formed CAF-antibody complexes from the biological material.

13. The method of claim 12 including the steps of:
- binding the antibody to an insoluble carrier
- exposure of body fluids such as blood to the bound antibody
- regeneration of the bound antibody by exposure to concentrated salt solutions and/ or low pH values

14. An immunological detection procedure for the detection of CAF of neuronal origin in biological material of human comprising in-vitro-treatment of a sample of said material with an antibody according to claim 11 and detecting CAF-antibody complexes.

15. Use of the monoclonal antibody according to claim 8 for the manufacture of a medicament for the treatment of diseases in which agrin or agrin cleavage is involved.

16. Use of the antibodies according to claim 8 for the detection of agrin or agrin fragments as marker or surrogate marker in clinical trials or as marker in personalized medicine.


SEQUENCE LISTING

<110>  Neurotune AG

<120>  A method for the production of hybridoma cell lines producing
       monoclonal antibodies capable to specifically binding to a human
       C44-fragment of agrin.

<130>  05478ep

<160>  13

<170>  PatentIn version 3.5


<210>  1

<211>  421

<212>  PRT

<213>  Artificial Sequence

<220>

<223>  Neurotrypsin resistant agrin fragment C44 y4z8

<400>  1

Leu Ala Asp Phe Asn Gly Phe Ser His Leu Glu Leu Arg Gly Leu His
1               5                   10                  15

Thr Phe Ala Arg Asp Leu Gly Glu Lys Met Ala Leu Glu Val Val Phe
            20                  25                  30

Leu Ala Arg Gly Pro Ser Gly Leu Leu Leu Tyr Asn Gly Gln Lys Thr
        35                  40                  45

Asp Gly Lys Gly Asp Phe Val Ser Leu Ala Leu Arg Asp Arg Arg Leu
    50                  55                  60

Glu Phe Arg Tyr Asp Leu Gly Lys Gly Ala Ala Val Ile Arg Ser Arg
65                  70                  75                  80

Glu Pro Val Thr Leu Gly Ala Trp Thr Arg Val Ser Leu Glu Arg Asn
                85                  90                  95

Gly Arg Lys Gly Ala Leu Arg Val Gly Asp Gly Pro Arg Val Leu Gly
            100                 105                 110

Glu Ser Pro Lys Ser Arg Lys Val Pro His Thr Val Leu Asn Leu Lys
        115                 120                 125
Glu Pro Leu Tyr Val Gly Gly Ala Pro Asp Phe Ser Lys Leu Ala Arg
    130                 135                 140
Ala Ala Ala Val Ser Ser Gly Phe Asp Gly Ala Ile Gln Leu Val Ser
145                 150                 155                 160
Leu Gly Gly Arg Gln Leu Leu Thr Pro Glu His Val Leu Arg Gln Val
                165                 170                 175
Asp Val Thr Ser Phe Ala Gly His Pro Cys Thr Arg Ala Ser Gly His
            180                 185                 190
Pro Cys Leu Asn Gly Ala Ser Cys Val Pro Arg Glu Ala Ala Tyr Val
        195                 200                 205
Cys Leu Cys Pro Gly Gly Phe Ser Gly Pro His Cys Glu Lys Gly Leu
    210                 215                 220
Val Glu Ala Ser Ala Gly Asp Val Asp Thr Leu Ala Phe Asp Gly Arg
225                 230                 235                 240
Thr Phe Val Glu Tyr Leu Asn Ala Val Thr Glu Ser Glu Leu Ala Asn
                245                 250                 255
Glu Ile Pro Val Glu Lys Ala Leu Gln Ser Asn His Phe Glu Leu Ser
            260                 265                 270
Leu Arg Thr Glu Ala Thr Gln Gly Leu Val Leu Trp Ser Gly Lys Ala
        275                 280                 285
Thr Glu Arg Ala Asp Tyr Val Ala Leu Ala Ile Val Asp Gly His Leu
    290                 295                 300
Gln Leu Ser Tyr Asn Leu Gly Ser Gln Pro Val Val Leu Arg Ser Thr
305                 310                 315                 320
Val Pro Val Asn Thr Asn Arg Trp Leu Arg Val Val Ala His Arg Glu
                325                 330                 335
Gln Arg Glu Gly Ser Leu Gln Val Gly Asn Glu Ala Pro Val Thr Gly
            340                 345                 350

Ser Ser Pro Leu Gly Ala Thr Gln Leu Asp Thr Asp Gly Ala Leu Trp
        355                 360                 365
Leu Gly Gly Leu Pro Glu Leu Pro Val Gly Pro Ala Leu Pro Lys Ala
    370                 375                 380
Tyr Gly Thr Gly Phe Val Gly Cys Leu Arg Asp Val Val Val Gly Arg
385                 390                 395                 400
His Pro Leu His Leu Leu Glu Asp Ala Val Thr Lys Pro Glu Leu Arg
                405                 410                 415
Pro Cys Pro Thr Pro
            420

<210>  2
<211>  4
<212>  PRT
<213>  Artificial Sequence
<220>
<223>  Insertion at the y-site of agrin (y4)
<400>  2
Lys Ser Arg Lys
1

<210>  3
<211>  8
<212>  PRT
<213>  Artificial Sequence
<220>
<223>  Insertion at the z-site of agrin (z8)
<400>  3
Glu Leu Ala Asn Glu Ile Pro Val
1               5

<210> 4

<211> 17

<212> PRT

<213> Artificial Sequence

<220>

<223> Insertion at the y-site of agrin (y17)

<400> 4

Val Leu Ser Ala Ser His Pro Leu Thr Val Ser Gly Ala Ser Thr Pro

1               5                   10                  15

Arg

<210> 5

<211> 21

<212> PRT

<213> Artificial Sequence

<220>

<223> Insertion at the y-site of agrin (y21

<400> 5

Lys Ser Arg Lys Val Leu Ser Ala Ser His Pro Leu Thr Val Ser Gly

1               5                   10                  15

Ala Ser Thr Pro Arg

            20

<210> 6

<211> 11

<212> PRT

<213> Artificial Sequence

<220>

<223>  Insertion at the z-site of agrin (z11)

<400>  6

Pro Glu Thr Leu Asp Ser Gly Ala Leu His Ser

1               5                   10

<210>  7

<211>  19

<212>  PRT

<213>  Artificial Sequence

<220>

<223>  Insertion at the z-site of agrin (z19)

<400>  7

Glu Leu Ala Asn Glu Ile Pro Val Pro Glu Thr Leu Asp Ser Gly Ala

1               5                   10                  15

Leu His Ser

<210>  8

<211>  5

<212>  PRT

<213>  Artificial Sequence

<220>

<223>  Epitope sequence

<400>  8

Thr Phe Val Glu Tyr

1               5

<210>  9

<211>  5

<212>  PRT

<213>  Artificial Sequence

<220>

<223>  Epitope Sequence

<400>  9

Phe Val Glu Tyr Leu

1               5

<210>  10

<211>  4

<212>  PRT

<213>  Artificial Sequence

<220>

<223>  Epitope Sequence

<400>  10

Thr Phe Val Glu

1

<210>  11

<211>  9

<212>  PRT

<213>  Artificial Sequence

<220>

<223>  Eptiope Sequence

<400>  11

Trp Leu Gly Gly Leu Pro Glu Leu Pro

1               5

<210> 12

<211> 5

<212> PRT

<213> Artificial Sequence

<220>

<223> Epitope Sequence

<400> 12

Leu Pro Glu Leu Pro

1               5

<210> 13

<211> 20

<212> PRT

<213> Artificial Sequence

<220>

<223> Amino acid sequence containing a His8 tag and a prescission

cleavage site

<400> 13

Ala Arg Val Asn His His His His His His His His Leu Glu Val Leu

1               5                   10                  15

Phe Gln Gly Pro

            20